# EUROPEAN PATENT SPECIFICATION

(11) **EP 1 912 683 B1**
(45) Date of publication and mention of the grant of the patent: **12.03.2014**
(21) Application number: 06800232.8
(22) Date of filing: 24.07.2006
(51) Int. Cl.: A61L 2/238, A61L 15/46, A61L 27/54, A61L 31/16, A01N 59/16, C08K 3/00, C08K 3/10, C08L 79/02

(54) **ANTIMICROBIAL COMPOSITION**
ANTIMIKROBIELLE ZUSAMMENSETZUNG
COMPOSITION ANTIMICROBIENNE

(30) Priority: 25.07.2005 US 188444
(43) Date of publication of application: 23.04.2008
(73) Proprietor: ETHICON, INC., Somerville, New Jersey 08876-0151 (US)
(72) Inventor: ROTHENBURGER, Stephen J., Neshanic Station, New Jersey 08853 (US); MING, Xintian, Bridgewater, New Jersey 08807 (US)
(74) Representative: James, Anthony Christopher W.P.
(86) International application number: PCT/US2006/028522
(87) International publication number: WO 2007/014087

(56) References cited:
- WO-A-95/34327
- WO-A1-2004/080499
- WO-A2-2004/056404
- US-A- 4 923 619
- US-A- 6 039 940
- US-A1- 2005 124 724
- US-B1- 6 468 521

## Description

### FIELD OF INVENTION

This invention relates to a stabilized antimicrobial composition comprising (a) polymeric polyquaternary ammonium compounds and (b) one or more antimicrobial metal; and to use of a polymeric polyquaternary ammonium compound for improving the stability of an antimicrobial composition comprising one or more antimicrobial metal. More speciflcally, this invention relates to a stabilized antimicrobial composition comprising polymeric polyquaternary ammonium compounds and specific silver compounds. The present invention also relates to wound dressing, surgical dressing and other medical devices utilising such stabilized antimicrobial compositions.

### BACKGROUND OF THE INVENTION

Each year, patients undergo a vast number of surgical procedures in the United States. Current data shows about twenty-seven million procedures are performed per year. Post-operative or surgical site infections ("SSIs") occur in approximately two to three percent of all cases. This amounts to more than 675,000 SSIs each year.

Whenever a medical device is used in a surgical setting, a risk of infection is created. The risk of infection dramatically increases for invasive or implantable medical devices, such as intravenous catheters, arterial grafts, intrathecal or intracerebral shunts and prosthetic devices, which create a portal of entry for pathogens while in intimate contact with body tissues and fluids. The occurrence of SSIs is often associated with bacteria that colonize on the medical device. For example, during a surgical procedure, bacteria from the surrounding atmosphere may enter the surgical site and attach to the medical device. Bacteria can use the implanted medical device as a pathway to surrounding tissue. Such bacterial colonization on the medical device may lead to infection and morbidity and mortality to the patient.

A number of methods for reducing the risk of infection associated with invasive or implantable medical devices have been developed that incorporate antimicrobial agents into the medical devices. Such devices desirably provide effective levels of antimicrobial agent while the device is being used. For example, medical devices may contain an antimicrobial agent such as silver.

The antimicrobial activity of silver compounds is a well known property which has been utilized for many years. More particularly, the antimicrobial effects are caused by silver ions that are released from, for example, silver compounds such as silver nitrate and silver sulfadiazine. Silver nitrate in concentrations of 0.5-1% (WN) in water shows disinfectant properties and is used for preventing infections in bums or for prophylaxis of neonatal conjunctivitis. Silver sulfadiazine is a silver complex, where both the sulfadiazine molecule and the silver ion have an antibacterial effect. Silver sulfadiazine is used intensively in the treatment of wounds, in particular for burns. Silver-protein-combinations are other antiseptic formulations which have been used in low concentrations, for example, in eye drops.

Antimicrobial agents based on silver compounds are also used in various medical devices. One example of such application is the use in the wound dressing sold by Johnson & Johnson under the trademark Actisorb®, which is an activated charcoal cloth dressing. Another example is the wound dressing sold under the trademark EZ-Derm by Genetic Laboratories, which is a modified pigskin impregnated with a soluble silver compound intended for treatment of bums. Additionally EP 272 149 B1 discloses a medical dressing of the "hydrocolloid" type containing, for example, silver chloride as an antiseptic compound.

A major drawback when using silver compounds that release silver ions for antimicrobial purposes is the dark stains that result on tissue or skin contacting the silver compound or the formulation or medical device having the silver compound incorporated thereon or therein. Such staining has been reported to give pigmentation of the skin, commonly referred to as argyria.

Although silver compounds are known to be efficacious antimicrobial agents, such compounds may also cause undesired changes in physical properties of formulations or medical devices having such silver compounds incorporated thereon or therein, both prior to and during use of the formulation or medical device. It is commonly recognized that formulations or medical devices containing silver compounds will discolor in the presence of an energy source, e.g. light and/or heat, or irradiation. For example, radiation sterilization can lead to an unsatisfactory change of color of a formulation such as a cream or a gel, or a medical device having a silver compound incorporated thereon or therein.

Photo-stable antimicrobial metal-based compositions have been disclosed in U.S. Pat. No 6, 468, 521, in which the silver compound is described as a complex between the silver ions and a primary, secondary or tertiary lower alkyl amine or amino alcohol. This reference discloses that this complex is stable in the presence of hydrophilic polymers during sterilization and retains its antimicrobial activity, without giving rise to darkening or discoloration of the dressing during storage. However, it is undesirable to utilize lower alkyl amines or amino alcohols for medical applications, since lower alkyl amines or amino alcohols generally are known to be irritants and moderately toxic compounds having numerous potential side effects. Furthermore, the lower alkyl amine itself is not an effective antimicrobial agent.

One potential solution to the problem posed by the use of a lower alkyl amine described in U.S. Pat. No 6, 468, 521, is the use of polymeric polyquaternary ammonium compounds and an antimicrobial metal to form a stabilized antimicrobial composition that is stable against discoloration upon exposure to light and/or heat and against the loss of antimicrobial activity.

Polymeric quaternary ammonium compounds are known to be less toxic and less irritating than monomeric amines. (Patty's Industrial Hygiene and Toxicology, Vol. II, Part B, 4th ed. 1994). An additional benefit of using polymeric polyquaternary ammonium compounds and an antimicrobial metal to form a stabilized antimicrobial composition is that polymeric quaternary ammonium compounds themselves are known to be effective antimicrobial agents. For instance, polyquatemium 1 demonstrates significant antimicrobial activity against a wide range of microorganisms, while lower alkyl amines such as monoethanolamines lack dramatic antimicrobial activity. (Disinfection, Sterilization, and Preservation 4th ed. 1991. S. Block ed. Lea & Febiger. pp 225, 232, 233, 313). Hence, the stabilized antimicrobial composition described herein possesses two active antimicrobial agent compared to the single antimicrobial agent described in U.S. Pat. No 6, 468, 521.

There have been no reports to date on the use of a combination of (a) polymeric polyquaternary ammonium compounds, and (b) an antimicrobial metal, which has been discovered to be stable upon exposure to light and/or heat, while possessing enhanced antimicrobial activity. More specifically, the use of the stabilized antimicrobial composition described herein alone, in a formulation, or in conjunction with a medical device, prevents the formation of dark colored sparingly soluble or insoluble silver compounds (*e.g. silver oxides)* and the resultant discoloration of the formulation or medical device having the antimicrobial composition incorporated thereon or therein.

### SUMMARY OF THE INVENTION

Described herein is a stabilized antimicrobial composition comprising (a) polymeric polyquatemary ammonium compound, and (b) one or more antimicrobial metal, in the form of at least one silver compound. The antimicrobial composition may be used as a stand-alone antimicrobial composition, in a formulation such as a cream or a gel, or in combination with medical articles or medical devices.

### DETAILED DESCRIPTION OF THE INVENTION

The present invention is directed to an antimicrobial composition as set out in claim 1 and to a use as set out in claim 3. More specifically, the present invention is directed to an antimicrobial composition comprising polymeric polyquaternary ammonium compounds and silver compounds, that may be used alone, in a formulation or in combination with medical devices to impart antimicrobial properties to the formulation or device.

The polymeric polyquaternary ammonium compounds described herein have the following formula (I): 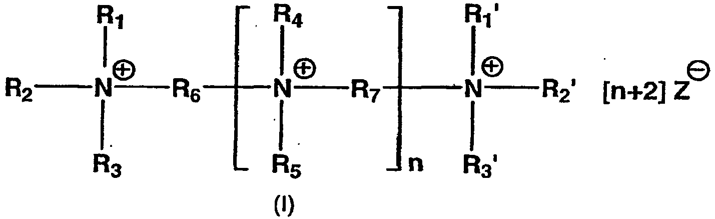 wherein R₁, R₂ , R₃, R_{1'}, R_{2'}, R_{3'}, R_{4,} R₅,, in the above formula may be identical or different, and are independently selected from hydrogen, an C₁-C₂₀ alkyl group, an aryl group, a benzyl group, an aralkyl group, or an alkylaryl group. Each C₁-C₂₀ alkyl group may be substituted or un-substituted, linear or branched. R₆, and R₇ in the above formula may be identical or different, and are independently selected from (CH₂)ₘ, or (CH₂)ₘ -(CH=CH)_{m'} -(CH₂)ₘ, wherein 12=>m>=1, 10=>m'>=1 and 150>=n=>5; and Z is anionic moiety selected from F, Cl, Br, And COOH.

The polyquatemium polymer compounds suitably have a weight average molecular weight M_{w} most preferably about 4600 to 11,000.

A particular example of such class of polyquaternium polymer compound is polyquaternium-1: α-4-[1-tris(2-hydroxyethyl) ammonium-2-butenyl] poly[1-dimethylammonium-2-butenyl]-ω-tris(2- hydroxyethyl)ammonium chloride (available under the trademark Onamer M^{®} from Onyx Chemical Company, Jersey City, N.J.; also known as Polyquad^{®}, a registered trademark of Alcon Laboratories, Inc., Ft. Worth, Tex.; also known as polyquaternium-1), having the chemical structure described in formula (II): 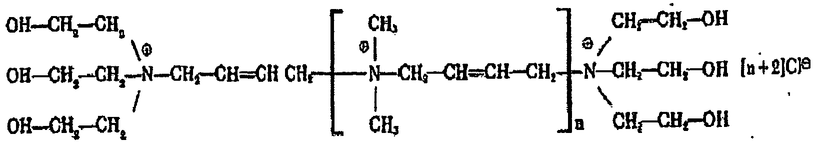 and may be made as the reaction product of 1, 4-bis[dimethylamino]-2-butene (0.9 mol), triethanolamine (0.2 mol), and 1, 4-dichloro-2-butene (1.0 mol) in water.

The polyquatemium polymer compounds may be in the form of a liquid concentrate, a salt, or a salt in aqueous solution. One particularly useful form of the polyquaternium polymer compounds is polyquaternium-1 chloride in aqueous solution.

Antimicrobial metals, in particular silver compounds, are especially potent against a broad spectrum of microorganisms. The silver compound referred to herein is a compound comprising a silver ion, linked to another molecule via a covalent or non-covalent linkage with the potential to be oxidized to form silver oxide. The silver compound of the present invention is selected from silver salts formed by silver lons with organic acids (e.g. acetic acids and fatty acids) or inorganic acids, specifically from silver sulfadiazine ("AgSD"), silver carbonate ("Ag ₂ CO ₃"), silver deoxycholate, silver salicylate, silver iodide, silver nitrate ("AgNO ₃"), silver paraaminobenzoate, silver paraaminosalicylate, silver acetylsalicylate, silver ethylenediaminetetraacetic acid ("Ag EDTA"), silver plcrate, silver protein, silver citrate, silver lactate, silver acetate and silver laurate.

In one particular set of non-limiting embodiments, the present invention provides an antimicrobial composition comprising a complex of polymeric polyquatemary ammonium compounds with one or more antimicrobial metal as described herein. The term "complex" as used herein refers to an intimate mixture at the molecular scale, preferably with ionic or covalent bonding between the antimicrobial metal and the polymeric polyquatemary ammonium compounds. The complex preferably comprises a salt formed between the polymeric polyquatemary ammonium compounds and ions of the antimicrobial metal, but it may also comprise metal clusters and/or colloidal metal, for example produced by exposure of the complex to light.

In one embodiment, the antimicrobial composition is in the form of an aqueous or organic solution of the polymeric polyquatemanry ammonium compound and the antimicrobial metal, which may be utilized in medical applications directly on tissue and skin, i.e., for the treatment of bums.

In another embodiment, the stabilized antimicrobial composition comprises (a) polymeric polyquatemary ammonium compounds (b) an antimicrobial metal as described herein, which may be incorporated into a formulation, independent of any medical devices or specific applications. Formulations of the antimicrobial composition may be of liquid (e.g. solutions) or solid form (e.g. powders), and may comprise the polymeric polyquaternary ammonium compound in an amount from about 0.001 % to about 5% by weight and the antimicrobial metal in an amount from about 0.001% to about 5 % by weight relative to total weight of the formulation.

More preferably, the formulation may comprise the polymeric polyquaternary ammonium compound in an amount from about 0.01% to about 1% by weight and the antimicrobial metal in an amount from about 0.01% to about 1% by weight relative to total weight of the formulation. For instance, formulations having the antimicrobial composition may be in form of cream or gel and may be applied directly to a wound.

In another set of non-limiting embodiments, the antimicrobial composition may be incorporated on or into medical devices. The terms "incorporate", "incorporated", or "incorporating", as used herein, refer to combining the composition with the medical device by physical or chemical means. Examples include, but are not limited to, impregnating, dipping, soaking or coating a medical device with an aqueous or organic solution of the antimicrobial composition or preparing the medical device by adding the antimicrobial composition to the material that the medical device is made from. The medical devices that may be treated are either fabricated from or coated or treated with a biomedical polymer and include, but are not limited to, microcapsules, dressings, implants, wound closures, staples, meshes, controlled drug delivery systems, wound coverings, fillers, sutures, tissue adhesives, tissue sealants, absorbable and non-absorbable hemostats, catheters including urinary catheters and vascular catheters (e.g., peripheral and central vascular catheters), wound drainage tubes, arterial grafts, soft tissue patches (such as polytetrafluoroethylene ("PTFE") soft tissue patches), gloves, shunts, stents, tracheal catheters, wound dressings, sutures, guide wires and prosthetic devices (e.g., heart valves and LVADs). The present invention may be further applied to medical articles that have been prepared according to U.S. Patent Nos. 3,839,297; 4,027,676; 4,185,637 and 4,201,216.

The medical dressings that may be treated are protein or polysaccharide based. Polysaccharide is selected from the group consisting of cellulose derivatives, chitin, chitosans, galactomannans, alginate and mixtures thereof. Protein is selected from the group consisting of collagen, gelatin and mixture thereof. Examples of such polysaccharide based dressings commercially available include Surgicel® absorbable hemostat; Surgicel Nu-Knit® absorbable hemostat; and Surgicel® Fibrillar absorbable hemostat; protein based dressings include Surgifoam® absorbable gelatin; and Promogran® dressing having collagen and oxidized regenerated cellulose, all available from Johnson & Johnson Wound Management Worldwide, a division of Ethicon, Inc., Somerville, New Jersey, a Johnson & Johnson Company. Where the medical article is a dressing, such as Surgicel® absorbable hemostat, a knitted fabric of oxidized regenerated cellulose (ORC), the amount of polymeric polyquaternary ammonium compound on the dressing may be from about 0.001-500 µg/cm², preferably from about 0.01-100 µg/cm², and the amount of silver metal may be from about 0.001-500 µg/cm², preferably 0.01-100 µg/cm². The term "about" as used herein indicates a variation within 20 percent.

The antimicrobial composition described herein is characterized by its stabilization effect to silver ions, thereby resulting in the prevention of discoloration of tissue or skin to which it is applied, or the formulation or medical device in which it is incorporated on or within. The use of this stabilized composition has also been shown to be effective against a broader antimicrobial spectrum of organisms including, but not limited, *Tinea pedis, Tinea unguium, Tinea cruris,* or *Tinea capitis, S. aureus, MRSA, MRSE, GISA, S. epidermidis, E. coli, P. aeruginosa, K. pneumoniae, B. cepacia, E. cloacae, S. marcescens, S. pyogenes, S. agalacticae, E. faecalis-Vancomycin Resistant, E faecium, C. albicans and B. subtilis, Salmonella sp., Proteus sp., Acinetobacter sp.Aspergillus niger.*

While the following examples demonstrate certain embodiments of the invention, they are not to be interpreted as limiting the scope of the invention, but rather as contributing to a complete description of the invention.

### Example 1

### Preparation of stabilized silver compound

Stock solutions of Ag NO₃ at 0.1 % and polyquaternium 1 (Onamer M, Stepan company, Northfield, IL USA) at 0.1 % were prepared in distilled water respectively. The silver-polyquat (Ag NO₃ - polyquaternium 1) complex was formed by mixing the two stock solutions at 1:1 ratio and was equilibrated for 24 hours at ambient temperature in the dark. The silver-polyquat complex solution was applied onto a white cellulose disc (1 cm diameter) at 100 ul /disc. The treated discs contained 100 ug silver nitrate (in the polyquat complex)/disc and was tested for light and heat stability as described in Example 5.

### Example 2

The silver- polyquat complex was applied to Surgicel®absorbable hemostat, a knitted fabric of oxidized regenerated cellulose (Ethicon Inc, a Johnson and Johnson company, Somerville NJ, USA). The Surgicel® absorbable hemostat was cut into 1 x 1 cm squares. The silver- polyquat complex solution prepared in Example 1 was applied onto the Surgicel® absorbable hemostat at 50 ul/ cm². Each Surgicel® absorbable hemostat section contained 50 ug silver nitrate (in the polyquat complex)/ cm² and was tested for light stability in Example 5.

### Example 3

The silver- polyquat complex was applied to Promogran® dressing, a collagen and oxidised regenerated cellulose product (Johnson & Johnson medical, Divison of Ethicon, Inc. Gargrave, U.K.). The Promogran® dressing was cut into 1 x 1 cm squares. The silver- polyquat complex solution prepared in Example 1 was applied onto the Promogran® dressing at 100 ul/ cm². Each Promogran® dressing section contained 100 ug silver nitrate (in the polyquat complex)/cm² and was tested for light stability in Example 5.

### Example 4

The silver-polyquat complex was applied to Nu-Gel® hydrogel (Johnson & Johnson medical, Divison of Ethicon, Inc. Gargrave, U.K.). The Nu-Gel® hydrogel was cut into 1 x 1 cm squares. The silver- polyquat complex solution prepared in Example 1 was applied onto the Nu-Gel® hydrogel section at 50 ul/cm². Each Nu-Gel® section contained 50ug silver nitrate (in the polyquat complex)/ cm² and was tested for light stability in Example 5.

### Example 5

Comparison of the stabilized silver compositions on devices with silver alone on devices against discoloration by heat and light.

Stability against light was determined by exposing samples from Examples 1-4 to sunlight through a glass window for 1 to 6 hours. Discoloration was determined by visual observation.

Stability against heat was determined for samples from Examples 1-4, which were each sealed into autoclavable bags and subjected to heat treatment at 121°C for 60 minutes.

Control samples ("Silver Alone" in Table 1) were prepared to contain the same amount of silver nitrate as the amount of silver nitrate in the polyquat complex, in each of the samples prepared in Example 1-4. The control samples where evaluated for stability against light and heat in the manner described above.

The use of the stabilized silver- polyquat complex prevented discoloration as illustrated by the results shown in Table 1. The control samples that contained 50 or 100ug Ag NO₃ became dark brown after one hour exposure to sunlight, while the samples of Example 1-4 containing 50ug or 100ug silver-polyquat complex showed no color change after more than 6 hours exposure of sunlight.

**Table 1. Silver stabilization against discoloration by using Polyquatemium 1 (Onamer M)**

| | | | | |
|---|---|---|---|---|
| | | | | |

| | Sunlight | | Heat | |
|---|---|---|---|---|
| Example | Silver Alone | Stabilized silver | Silver Alone | Stabilized silver |
| 1 | dark brown | white | brown | white |
| 2 | yellow brown | white | N/A | N/A |
| 3 | dark brown | white | N/A | N/A |
| 4 | dark brown | white | N/A | N/A |

### Example 6

Comparison of the antimicrobial efficacy of stabilized silver compositions on samples with samples having silver alone, after heat treatment and sunlight exposure

The sample of Example 1 that was tested in Example 5 was further evaluated for its antimicrobial efficacy. The samples were placed into Tryptic Soy agar medium inoculated with about 10⁵ cfu bacteria. Zones of inhibition were recorded after the plates were incubated at 37°C for 24 hr. Zone of inhibition was defined as the distance from edge of the sample to the clear edge of bacterial lawn.

The results in Table 2 indicate that after exposure to sunlight or heat, the articles treated with the stabilized silver composition preserved theantimicrobial efficacy, while the control samples treated with silver alone showed reduced efficacy by the same exposure. This effect was shown by the result of zone of inhibition against *E*. *coli* and *E. faecium.* No zone of inhibition against *E*. *faecium* was observed on samples containing AgNO₃ alone upon sunlight or heat exposure, which indicates a significant loss in efficacy by sunlight and heat exposure. The samples containing the stabilized silver composition showed.no efficacy loss by the same exposure, indicated by similar zones of inhibition before and after the exposure.

**Table 2.**

| | | Zone of inhibition (mm) | | |
|---|---|---|---|---|
| **Exposure** | | **Discoloration** | ***E. coli*** | ***E. faecium*** |
| Without exposure | | | | |
| 100 ug AgNO₃ on disc | | white | 3.2 | 3.0 |
| | (Prepared as "Silver Alone" in Example 5) | | | |
| 100 ug Polyquat on disc | | white | 0 | 0 |
| 100 ug AgNO₃ on disc | | white | 3.8 | 3.6 |
| | (in the silver poylquat complex and prepared in Example 1) | | | |
| | | | | |

| Sunlight | | | | |
|---|---|---|---|---|
| 100 ug AgNO₃ on disc | | dark brown | 2.1 | 0 |
| | (Prepared as "Silver Alone" in Example 5) | | | |
| 100 ug Polyquat on disc | | white | 0 | 0 |
| 100 ug AgNO₃ on disc | | white | 3.7 | 3.4 |
| | (in the silver poylquat complex and prepared in Example 1) | | | |
| | | | | |

| Heat | | | | |
|---|---|---|---|---|
| 100 ug AgNO₃ on disc | | dark brown | unclear zone | 0 |
| | (Prepared as "Silver Alone" in Example 5) | | | |
| 100 ug Polyquat on disc | | white | 0 | 0 |
| 100 ug AgNO₃ on disc | | white | 2.8 | 2.2 |
| | (in the silver polyquat complex and prepared in Example 1) | | | |

### Example 7

Comparison of stabilized silver- polyquat with stabilized silver- monomeric amines against discoloration by light.

Stock solutions of AgNO₃ at 0.1 %, tri-hydroxymethyl-aminomethane (Tris, Sigma) at 0.1 %, and polyquatemium 1 (Onamer M, Stepan company, Northfield, IL USA) at 0.1 % were prepared in distilled water respectively. The silver-stabilized complexes were formed by mixing the silver nitrate stock solutions with either the polyquatemium 1 stock solution or the tri-hydroxymethyl-aminomethane stock solution at 1:1 ratio and were equilibrated for 24 hours at ambient temperature in the dark. The silver-stablized complex solutions with either polyquatemium 1 or tri-hydroxymethyl-aminomethane were applied onto white cellulose disc (1 cm diameter) at 100 ul /disc. Silver nitrate alone was applied onto a separate white cellulose disc (1 cm diameter) at 100 ul /disc. All discs were exposed to sunlight for 1 hr and the discoloration was observed and recorded.

**Table 3**

| Sample | | discoloration by sunlight exposure |
|---|---|---|
| 100 ug AgNO₃ on disc | | dark brown |
| | (Prepared as "Silver Alone" in Example 5) | |
| 100 ug AgNO₃ on disc | | light brown |
| | (in the silver Tris complex and prepared in Example 1) | |
| 100 ug AgNO₃ on disc | | white |
| | (in the silver polyquat complex and prepared in Example 1) | |

## Claims

1. An antimicrobial composition in the form of a cream or a gel for application directly to a wound, said composition comprising:
a polymeric polyquaternary ammonium compound, according to the following formula: 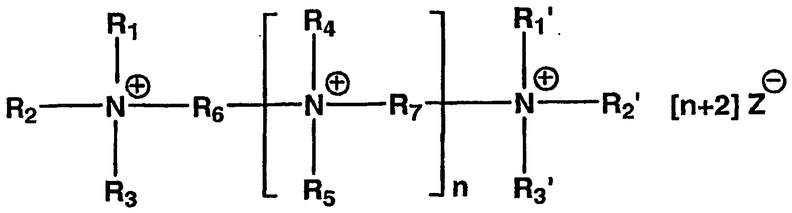 wherein R₁, R₂, R₃, R_{1',} R_{2'}, R_{3'}, R₄, R₅, in the above formula may be identical or different, and are independently selected from hydrogen, an C₁-C₂₀ alkyl group, an aryl group, a benzyl group, an aralkyl group, or an alkylaryl group, and each C₁-C₂₀ alkyl group may be substituted or unsubstituted, linear or branched; R₆, and R₇ in the above formula may be identical or different, and are independently selected from the group consisting of (CH₂)ₘ, and (CH₂)ₘ-(CH=CH)_{m'}-(CH₂)ₘ; wherein 12≥m≥1, 10≥m'≥1 and 150≥n≥5; and Z is anionic moiety selected from the group consisting of F, Cl, Br, I and COOH;
and an antimicrobial metal in the form of at least one silver compound, wherein said composition comprises from 0.01% to 1% by weight of said polymeric polyquaternary ammonium compound and from 0.01% to 1% by weight of silver, based on the total weight of the composition, and wherein said at least one silver compound is selected from the group consisting of silver sulfadiazine ("AgSD"), silver carbonate ("Ag₂CO₃"), silver deoxycholate, silver salicylate, silver iodide, silver nitrate ("AgNO₃"), silver paraaminobenzoate, silver paraaminosalicylate, silver acetylsalicylate, silver ethylenediaminetetraacetic acid ("Ag EDTA"), silver picrate, silver protein, silver citrate, silver lactate, silver acetate and silver laurate.

2. The composition of claim 1, wherein the polymeric polyquaternary ammonium compound is polyquaternium-1, according to the following formula:

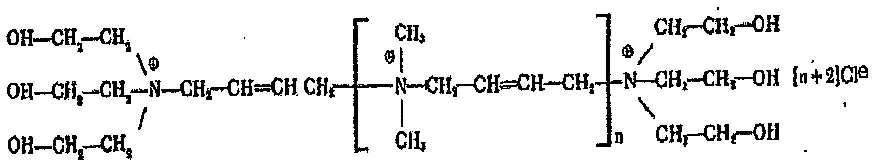

3. Use of a polymeric polyquaternary ammonium compound, according to the following formula: 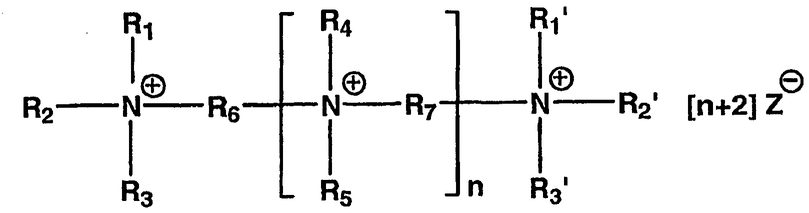 wherein R₁, R₂, R₃, R_{1'}, R_{2'}, R_{3'}, R₄, R₅, in the above formula may be identical or different, and are independently selected from hydrogen, an C₁-C₂₀ alkyl group, an aryl group, a benzyl group, an aralkyl group, or an alkylaryl group, and each C₁-C₂₀ alkyl group may be substituted or unsubstituted, linear or branched; R₆, and R₇ in the above formula may be identical or different, and are independently selected from the group consisting of (CH₂)ₘ, and (CH₂)ₘ-(CH=CH)ₘ-(CH₂)ₘ; wherein 12≥m≥1, 10≥m'≥1 and 150≥n≥5; and Z is anionic moiety selected from the group consisting of F, Cl, Br, I and COOH;
for improving the heat and/or light stability of an antimicrobial composition comprising at least one antimicrobial silver compound and the said polymeric polyquaternary ammonium compound, wherein said at least one antimicrobial silver compound is selected from the group consisting of silver sulfadiazine ("AgSD"), silver carbonate ("Ag₂CO₃"), silver deoxycholate, silver salicylate, silver iodide, silver nitrate ("AgNO₃"), silver paraaminobenzoate, silver paraaminosalicylate, silver acetylsalicylate, silver ethylenediaminetetraacetic acid ("Ag EDTA"), silver picrate, silver protein, silver citrate, silver lactate, silver acetate and silver laurate.

4. Use according to claim 3 wherein the composition is incorporated on or into a medical device.

5. Use according to claim 3, wherein said composition comprises from 0.01% to 1% by weight of said polymeric polyquaternary ammonium compound and from 0.01% to 1% by weight of silver, based on the total weight of the composition.

6. Use according to claim 3, wherein the polymeric polyquaternary ammonium compound is polyquaternium-1, according to the following formula:

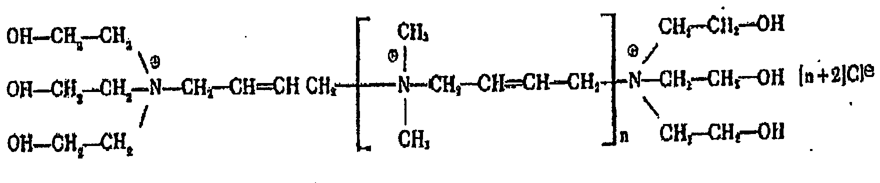

## Patentansprüche

1. Antimikrobielle Zusammensetzung in Form einer Creme oder eines Gels zur direkten Auftragung auf eine Wunde, wobei die Zusammensetzung Folgendes umfasst:
eine polymere polyquaternäre Ammoniumverbindung gemäß der folgenden Formel: 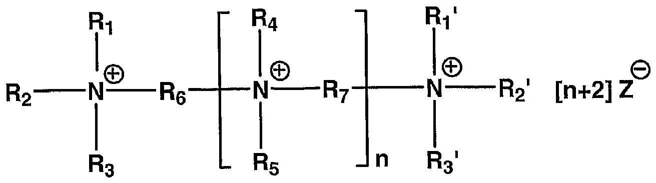 wobei R₁, R₂, R₃, R_{1'}, R_{2'}, R_{3'}, R₄, R₅ in der obigen Formel gleich oder verschieden sein können und unabhängig aus Wasserstoff, einer C₁-C₂₀-Alkylgruppe, einer Arylgruppe, einer Benzylgruppe, einer Aralkylgruppe oder einer Alkylarylgruppe ausgewählt sind und die C₁-C₂₀-Alkylgruppe jeweils substituiert oder unsubstituiert, linear oder verzweigt sein kann; R₆ und R₇ in der obigen Formel gleich oder verschieden sein können und unabhängig aus der aus (CH₂)ₘ und (CH₂)ₘ- (CH=CH)ₘ'- (CH₂)ₘ bestehenden Gruppe ausgewählt sind; wobei gilt: 12≥m≥1, 10≥m'≥1 und 150≥n≥5; und Z für eine aus der aus F, Cl, Br, I und COOH bestehenden Gruppe ausgewählte anionische Gruppierung steht;
und ein antimikrobielles Metall in Form wenigstens einer Silberverbindung, wobei die Zusammensetzung 0,01 Gew.-% bis 1 Gew.-% der polymeren polyquaternären Ammoniumverbindung und 0,01 Gew.-% bis 1 Gew.-% Silber bezogen auf das Gesamtgewicht der Zusammensetzung umfasst und wobei die wenigstens eine Silberverbindung aus der aus Silbersulfadiazin ("AgSD"), Silbercarbonat ("Ag₂CO₃"), Silberdesoxycholat, Silbersalicylat, Silberiodid, Silbernitrat ("AgNO₃"), Silberparaaminobenzoat, Silberparaaminosalicylat, Silberacetylsalicylat, Silber-Ethylendiamintetraessigsäure ("Ag-EDTA"), Silberpikrat, Silber-Protein, Silbercitrat, Silberlactat, Silberacetat und Silberlaurat bestehenden Gruppe ausgewählt ist.

2. Zusammensetzung nach Anspruch 1, wobei es sich bei der polymeren polyquaternären Ammoniumverbindung um Polyquaternium-1 gemäß der folgenden Formel handelt:

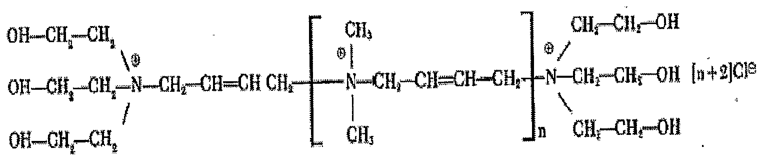

3. Verwendung einer polymeren polyquaternären Ammoniumverbindung gemäß der folgenden Formel: 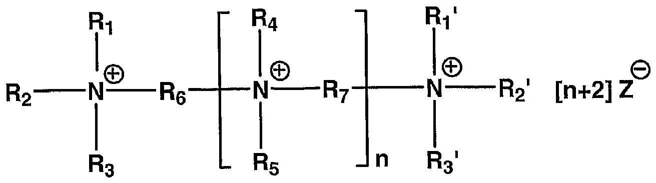 wobei R₁, R₂, R₃, R_{1'}, R_{2'}, R_{3'}, R₄, R₅ in der obigen Formel gleich oder verschieden sein können und unabhängig aus Wasserstoff, einer C₁-C₂₀-Alkylgruppe, einer Arylgruppe, einer Benzylgruppe, einer Aralkylgruppe oder einer Alkylarylgruppe ausgewählt sind und die C₁-C₂₀-Alkylgruppe jeweils substituiert oder unsubstituiert, linear oder verzweigt sein kann; R₆ und R₇ in der obigen Formel gleich oder verschieden sein können und unabhängig aus der aus (CH₂)ₘ und (CH₂)ₘ- (CH=CH)_{m'}- (CH₂)ₘ bestehenden Gruppe ausgewählt sind; wobei gilt: 12≥m≥1, 10≥m'≥1 und 150≥n≥5; und Z für eine aus der aus F, Cl, Br, I und COOH bestehenden Gruppe ausgewählte anionische Gruppierung steht; zur Verbesserung der Hitze- und/oder Lichtstabilität einer wenigstens eine antimikrobielle Silber-verbindung und die polymere polyquaternäre Ammoniumverbindung umfassenden antimikrobiellen Zusammensetzung, wobei die wenigstens eine antimikrobielle Silberverbindung aus der aus Silbersulfadiazin ("AgSD"), Silbercarbonat ("Ag₂CO₃"), Silberdesoxycholat, Silbersalicylat, Silberiodid, Silbernitrat ("AgNO₃"), Silberparaaminobenzoat, Silberparaaminosalicylat, Silberacetylsalicylat, Silber-Ethylendiamintetraessigsäure ("Ag-EDTA"), Silberpikrat, Silber-Protein, Silbercitrat, Silberlactat, Silberacetat und Silberlaurat bestehenden Gruppe ausgewählt ist.

4. Verwendung gemäß Anspruch 3, wobei die Zusammensetzung auf oder in eine medizinische Vorrichtung eingebaut ist.

5. Verwendung gemäß Anspruch 3, wobei die Zusammensetzung 0,01 Gew.-% bis 1 Gew.-% der polymeren polyquaternären Ammoniumverbindung und 0,01 Gew.-% bis 1 Gew.-% Silber bezogen auf das Gesamtgewicht der Zusammensetzung umfasst.

6. Verwendung gemäß Anspruch 3, wobei es sich bei der polymeren polyquaternären Ammoniumverbindung um Polyquaternium-1 gemäß der folgenden Formel handelt:

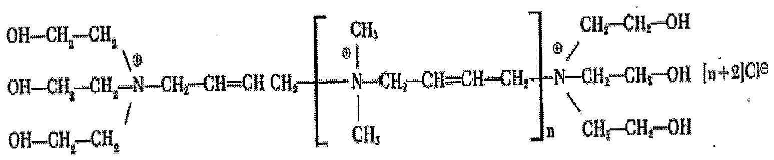

## Revendications

1. Composition antimicrobienne sous la forme d'une crème ou d'un gel pour une application directement à une plaie, ladite composition comprenant :
un composé d'ammonium poly-quaternaire polymère, répondant à la formule suivante : 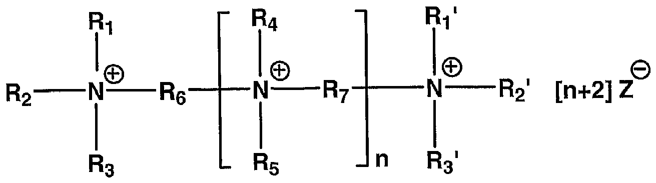 dans laquelle R₁, R₂, R₃, R₁, R_{2'}, R_{3'}, R₄ et R₅ dans la formule ci-dessus peuvent être identiques ou différents, et sont choisis indépendamment parmi hydrogène, un groupement C₁-C₂₀ alkyle, un groupement aryle, un groupement benzyle, un groupement aralkyle, ou un groupement alkylaryle, et chaque groupement C₁-C₂₀ alkyle peut être substitué ou non substitué, linéaire ou ramifié ; R₆ et R₇ dans la formule ci-dessus peuvent être identiques ou différents, et sont choisis indépendamment dans le groupe constitué par (CH₂)ₘ, et (CH₂)m- (CH=CH)ₘ- (CH₂)ₘ, où 12 ≥ m ≥ 1, 10 ≥ m ≥ 1 et 150 ≥ n ≥ 5 ; et Z est un motif anionique choisi dans le groupe constitué par F, Cl, Br, I et COOH ;
et un métal antimicrobien sous la forme d'au moins un composé d'argent, où ladite composition comprend de 0,01% à 1% en poids dudit composé d'ammonium poly-quaternaire polymère et de 0,01% à 1% en poids d'argent, sur la base du poids total de la composition, et où ledit au moins un composé d'argent est choisi dans le groupe constitué par la sulfadiazine d'argent (« AgSD »), le carbonate d'argent (« Ag₂CO₃ »), le désoxycholate d'argent, le salicylate d'argent, l'iodure d'argent, le nitrate d'argent (« AgNO₃ »), le para-aminobenzoate d'argent, le para-aminosalicylate d'argent, l'acétylsalicylate d'argent, l'acide éthylènediaminetétraacétique d'argent (« Ag EDTA »), le picrate d'argent, le protéinate d'argent, le citrate d'argent, le lactate d'argent, l'acétate d'argent et le laurate d'argent.

2. Composition selon la revendication 1, dans laquelle le composé d'ammonium poly-quaternaire polymère est le poly-quaternium-1, répondant à la formule suivante :

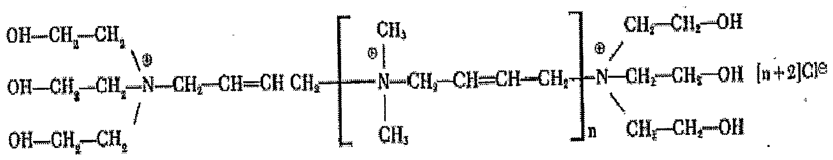

3. Utilisation d'un composé d'ammonium poly-quaternaire polymère, répondant à la formule suivante : 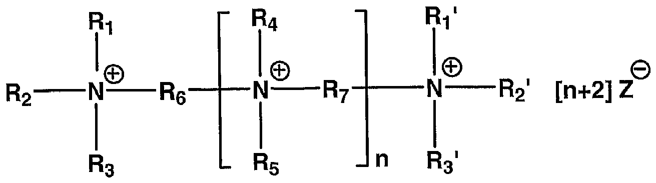 dans laquelle R₁, R₂, R₃, R_{1'}, R_{2'}, R_{3'}, R₄ et R₅ dans la formule ci-dessus peuvent être identiques ou différents, et sont choisis indépendamment parmi hydrogène, un groupement C₁-C₂₀ alkyle, un groupement aryle, un groupement benzyle, un groupement aralkyle, ou un groupement alkylaryle, et chaque groupement C₁-C₂₀ alkyle peut être substitué ou non substitué, linéaire ou ramifié ; R₆ et R₇ dans la formule ci-dessus peuvent être identiques ou différents, et sont choisis indépendamment dans le groupe constitué par (CH₂)ₘ, et (CH₂)ₘ-(CH=CH)ₘ-(CH₂)ₘ, où 12 ≥ m ≥ 1, 10 ≥ m ≥ 1 et 150 ≥ n ≥ 5 ; et Z est un motif anionique choisi dans le groupe constitué par F, Cl, Br, I et COOH ;
pour améliorer la stabilité vis-à-vis de la chaleur et/ou de la lumière d'une composition antimicrobienne, comprenant au moins un composé d'argent antimicrobien, et ledit composé d'ammonium poly-quaternaire polymère, où ledit au moins un composé d'argent antimicrobien est choisi dans le groupe constitué par la sulfadiazine d'argent (« AgSD »), le carbonate d'argent (« Ag₂CO₃ »), le désoxycholate d'argent, le salicylate d'argent, l'iodure d'argent, le nitrate d'argent (« AgNO₃ »), le para-aminobenzoate d'argent, le para-aminosalicylate d'argent, l'acétylsalicylate d'argent, l'acide éthylènediaminetétraacétique d'argent (« Ag EDTA »), le picrate d'argent, le protéinate d'argent, le citrate d'argent, le lactate d'argent, l'acétate d'argent et le laurate d'argent.

4. Utilisation selon la revendication 3, dans laquelle la composition est incorporée sur ou dans un dispositif médical.

5. Utilisation selon la revendication 3, dans laquelle ladite composition comprend de 0,01% à 1% en poids dudit composé d'ammonium poly-quaternaire polymère et de 0,01% à 1% en poids d'argent, sur la base du poids total de la composition.

6. Utilisation selon la revendication 3, dans laquelle le composé d'ammonium poly-quaternaire polymère est le poly-quaternium-1, répondant à la formule suivante :
